# EUROPEAN PATENT APPLICATION

(11) **EP 1 391 217 A1**
(43) Date of publication of application: **25.02.2004**
(21) Application number: 03394069.3
(22) Date of filing: 29.07.2003
(51) Int. Cl.: A61M 25/01

(54) **Over-the-wire catheter having a slidable instrument for gripping a guidewire**

(30) Priority: 21.08.2002 US 226789
(71) Applicant: Ave Connaught, Earlsfort Terrace, Dublin 2 (IE)
(72) Inventor: Carmody, Partick J., College Road, Galway (IE); Duane, Patrick J. Medtronic Vascular Inc., Galway (IE)
(74) Representative: McKeown, Yvonne Mary

(57) **Abstract**

An over-the-wire catheter having a guidewire lumen with a guide way extending along a shaft portion, and a slidable instrument disposed about the shaft portion and extending transversely through the guide way for surrounding and selectively gripping a guidewire disposed in the guidewire lumen. The slidable instrument may be operated to control the location of the guidewire within a patient.

## Description

### FIELD OF THE INVENTION

The present invention relates to catheters used with guidewires in the cardiovascular system and, in particular, to a catheter having a slidable instrument for facilitating exchange of such catheters and guidewires, and for advancing such catheters and guidewires to selected sites within a patient.

### BACKGROUND OF THE INVENTION

Catheters are inserted to various locations within a patient for a wide variety of purposes and medical procedures. For example only, one type of catheter is used in percutaneous catheter intervention (PCI) for the treatment of a vascular constriction termed a stenosis. In this instance, the catheter has a distally mounted balloon that can be placed, in a deflated condition, within the stenosis, and then inflated to dilate the narrowed lumen of the blood vessel. Such balloon dilation therapy is generally named percutaneous transluminal angioplasty (PTA). The designation PTCA, for percutaneous transluminal coronary angioplasty, is used when the treatment is more specifically employed in vessels of the heart. PTCA is used to open coronary arteries that have been occluded by a build-up of cholesterol fats or atherosclerotic plaque. The balloon at the distal end of the catheter is inflated, causing the site of the stenosis to widen.

The dilation of the occlusion, however, can form flaps, fissures and dissections, which may result in reclosure of the dilated vessel or even perforations in the vessel wall. Implantation of a stent can provide support for such flaps and dissections and thereby prevent reclosure of the vessel or provide a patch repair for a perforated vessel wall until corrective surgery can be performed. A stent is typically a cylindrically shaped device formed from wire(s) or a metal tube and is intended to act as a permanent prosthesis. A stent is deployed in a body lumen from a radially compressed configuration into a radially expanded configuration that allows it to contact and support a body lumen. A stent can be implanted during an angioplasty procedure by using a balloon catheter bearing a compressed stent that has been loaded onto the balloon. The stent radially expands as the balloon is inflated, forcing the stent into contact with the body lumen, thereby forming a supporting relationship with the lumen walls. Alternatively, self-expanding stents may be deployed with a sheath-based delivery catheter. Deployment is effected after the stent has been introduced percutaneously, transported transluminally and positioned at a desired location by the delivery catheter. In addition to angioplasty and stenting procedures, other therapeutic procedures require use of a delivery catheter, such as drug delivery, filters, occlusion devices, diagnostic devices and radiation treatment.

Typically, the placement of such therapeutic delivery catheters involves the use of a guidewire, which may be inserted into the patient's vasculature through the skin, and advanced to the location of the treatment site. The delivery catheter, which has a lumen adapted to receive the guidewire, then is advanced over the guidewire. Alternatively, the guidewire and the delivery catheter may be advanced together, with the guidewire protruding from the distal end of the delivery catheter. In either case, the guidewire serves to guide the delivery catheter to the location to be treated.

To treat small diameter vessels remote from the entry point into the patient, a guiding catheter is used to span the distance. For example, in PTCA or stent delivery, a guiding catheter is typically inserted into a large artery near the patient's groin, and then advanced toward the heart to the entry opening, or ostium, of the diseased coronary artery. The guiding catheter provides a tubular conduit through which catheters and guidewires can be passed from outside the patient to the vessel being treated.

There are three general types of catheters: "over-the-wire" (OTW) catheters, "rapid exchange" (RX) or single operator catheters and "fixed wire" (FW) or "balloon-on-a-wire" catheters. An over-the-wire catheter comprises a guidewire lumen that extends the entire length of the catheter. The guidewire is disposed entirely within the catheter guidewire lumen except for the distal and proximal guidewire portions, which extend beyond the distal and proximal ends of the catheter respectively. An OTW catheter typically has a "co-axial" catheter construction, wherein two hollow tubes are nested together such that lumen 17 of the inner tube can slidably receive guidewires and annular luminal space 19 formed between the inner and outer tubes is used for inflation/deflation fluid, as shown in FIGS. 1 and 2. An alternative "multi lumen" OTW catheter construction has an elongate shaft made from a single extruded tube having two lumens 17' and 19' formed side-by-side, as shown in FIGS. 3 and 4. OTW catheters that contain both multi lumen segments and coaxial segments are also known.

Over-the-wire catheters have many advantages traceable to the presence of a full-length guidewire lumen. Some of these advantages are good stiffness and pushability for readily advancing the catheter through tortuous vasculature and across tight stenoses. The full-length guidewire lumen is also available for transporting radiocontrast dye to the stenosed artery, for making pressure measurements, for infusing drugs, and for other therapies. Finally, the full-length guidewire lumen permits removal and replacement of a guidewire in an indwelling catheter, as may be required to alter the shape of the guidewire tip. It is also sometimes desirable to exchange one guidewire for another guidewire having a different stiffness. For example, a relatively soft, or flexible guidewire may prove to be suitable for guiding a PTCA catheter through a particularly tortuous anatomy, whereas following up with a stent-delivery catheter through the same vasculature region may require a guidewire that is relatively stiffer.

Over-the-wire catheters do suffer some shortcomings, however. For example, it often becomes necessary, in the performance of a PCI, to exchange one indwelling catheter for another catheter. In order to maintain a guidewire in position while withdrawing the catheter, the guidewire must be gripped at its proximal end to prevent it from being pulled out of the blood vessel with the catheter. For example, a PTCA catheter, which may typically be on the order of 135 centimeters long, is longer than the proximal portion of the standard guidewire that protrudes out of the patient. Therefore, exchanging an over-the-wire PTCA catheter requires an exchange guidewire of about 300 centimeters long, whereas a standard guidewire is about 165 centimeters long.

In one type of over-the-wire catheter exchange, the standard length guidewire first is removed from the lumen of the indwelling catheter. Then, a longer exchange guidewire is passed through the catheter to replace the original wire. Next, while holding the exchange guidewire by its proximal end to control its position in the patient, the catheter is withdrawn proximally from the blood vessel over the exchange guidewire. After the first catheter has been removed, the next OTW catheter is threaded onto the proximal end of the exchange guidewire and is advanced along the exchange guidewire, through the guiding catheter, and into the patient's blood vessels until the distal end of the catheter is at the desired location. The exchange guidewire may be left in place or it may be exchanged for a shorter, conventional-length guidewire. In an alternative type of catheter exchange procedure, the length of the initial guidewire may be extended by way of a guidewire extension apparatus. Regardless of which exchange process is used, the very long exchange guidewire is awkward to handle, thus requiring at least two operators to perform the procedure.

Catheter designs have been developed in an attempt to eliminate the need for guidewire extensions or exchange guidewires. One such catheter design is the rapid exchange (RX) type catheter. Catheters of this type are formed so that the guidewire is located outside of the catheter except for a short guidewire lumen that extends within only a comparatively short distal segment of the catheter. The rapid exchange catheter's proximal exit port for the guidewire is typically located about 5 cm (2.0 in) to 30 cm (11.8 in) proximal to the catheter's distal end. In use, the guidewire is placed initially in the patient's vascular system. The distal segment of the RX catheter then is threaded onto the wire. The catheter can be advanced alongside the guidewire with its distal segment being attached to and guided along the guidewire. The RX catheter can be removed and exchanged for another RX catheter without the use of a very long exchange guidewire and without requiring withdrawal of the initially placed guidewire.

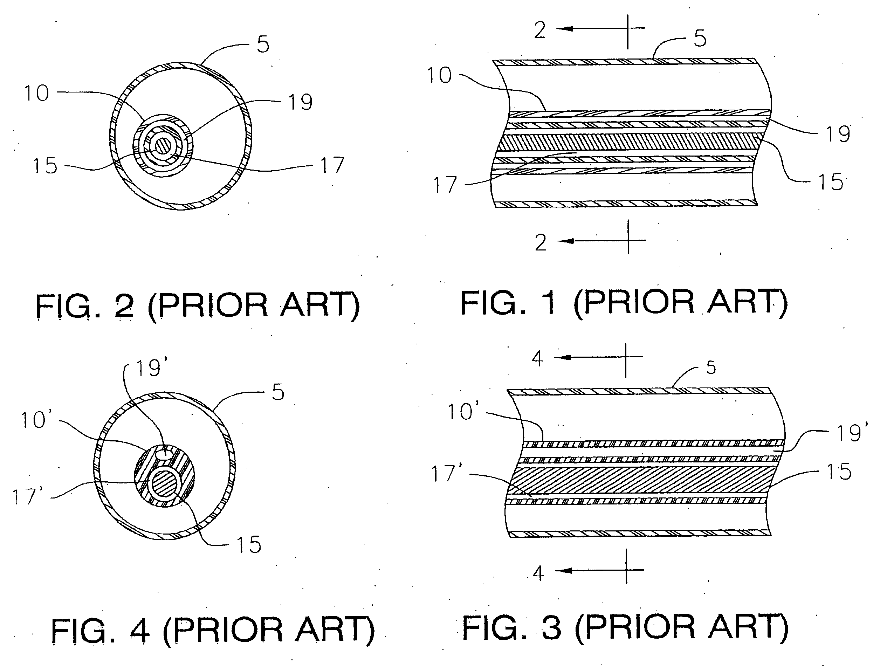

Although an RX catheter system may avoid the requirement for using a very long exchange wire, it presents several difficulties. First, without a full-length guidewire lumen, the proximal shaft of an RX catheter lacks an OTW catheter's coaxial interrelationship with the guidewire, which provides optimal transmission of force to push the distal end of the catheter through tight stenoses and/or tortuous blood vessels. FIGS. 1 and 2 illustrate guiding catheter 5, a shaft segment of OTW catheter 10 extending there through, and guidewire 15 disposed within guidewire lumen 17 in the common construction of coaxial tubes. The nested tubes result in an inner guidewire lumen 17 and an annular inflation lumen 19 formed between the tubes. The coaxial interrelationship with guidewire 15 provides an optimal transmission of force along the length of catheter 10. In FIGS. 3 and 4, inflation lumen 19' extends parallel to guidewire lumen 17' in a side-by-side arrangement. Although guidewire lumen 17' and guidewire 15 are located off-center in catheter 10', guidewire 15 is confined within catheter 10' throughout its length. Even if catheter 10' begins to buckle slightly when the distal tip of the catheter is being forced through a tight stenosis, there is very little misalignment with guidewire 15, such that most of the push force is transmitted to the distal tip. Therefore, despite their disadvantages during catheter exchange procedures, OTW catheters remain popular in the United States, due in part to the coaxial alignment between the catheter shaft and the guidewire, and the resulting excellent pushability of the device.

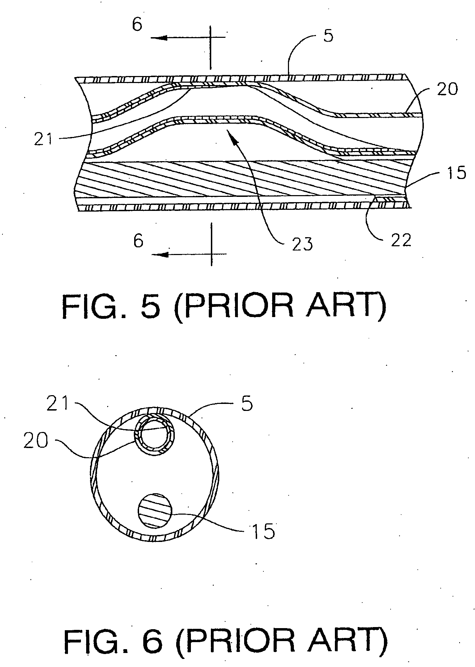

While improvements to RX catheters have incorporated stiff, metal proximal shafts and axial overlap between the shaft and the guidewire lumen to overcome the deficiencies discussed above, such RX catheters still are not optimal. FIG. 5 depicts prior art RX catheter 20 incorporating such a reinforced shaft 21, disposed over guidewire 15 within guiding catheter 5. However, even with continuous column support of the proximal shaft, the non-aligned or offset arrangement of guidewire 15 and shaft 21 of catheter 20, as illustrated in FIG. 6, can cause shaft buckling within the guiding catheter, as illustrated generally in FIG. 5, especially when the distal tip of the catheter is being forced through a tight stenosis. Such a non-coaxial misalignment causes displacement of push forces and an associated resistance to catheter advancement, especially in the region of proximal guidewire port 22.

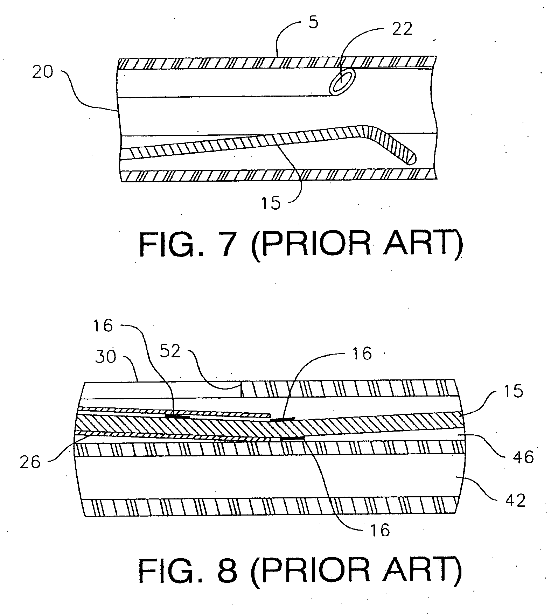

A second difficulty associated with RX catheters is that it is not possible to exchange guidewires in an indwelling RX catheter, as can be done advantageously with OTW catheters. A guidewire can be withdrawn, sometimes unintentionally, from the proximal guidewire port, thus derailing an indwelling RX catheter. However, neither the first guidewire nor a replacement guidewire can be directed back into the catheter's proximal guidewire port, which is hidden remotely in the guiding catheter within the patient. FIG. 7 illustrates the problem of blindly steering the tip of guidewire 15 within guiding catheter 5 in an attempt to find and engage proximal guidewire port 22 of RX catheter 20.

A third difficulty associated with RX catheters is that, if the guidewire lumen is so short that the proximal guidewire port exits from the distal end of the guiding catheter, then the guidewire will be exposed. Such an RX device presents a risk of what is called the "cheese cutter effect," which is damage to the delicate inner surface of a curved artery from straightening tension applied to the exposed guidewire during push-pull maneuvers to advance the catheter. The short-lumen RX device also presents an increased risk of guidewire entanglement in those procedures where multiple guidewires are used, because the guidewires are exposed within the blood vessel. Furthermore, the exposed, unprotected portion of the guidewire can become kinked or tangled within the patient's vessel, adding complications to the procedure.

A fourth difficulty associated with RX catheters is encountered at the proximal end of the catheter system. There, the RX catheter and the guidewire extend from the guiding catheter side-by-side, making it awkward to seal the system against blood loss during manipulation of the components. The sealing, or "anti-backbleed" function is typically accomplished with a "Tuohy-Borst" fitting that has a manually adjustable gasket with a round center hole that does not conform well to the side-by-side arrangement of a catheter shaft and guidewire. A final difficulty associated with RX catheters is that the lack of a full-length guidewire lumen deprives the clinician of an additional lumen that may be used for other purposes, such as pressure measurement, injection of contrast dye distal to the stenosis, or infusing a drug.

A catheter designed to eliminate the need for guidewire extensions or exchange wires is disclosed in U.S. Pat. No. 4,988,356 (Crittenden *et al.).* This over-the-wire/short wire or "otw/sw" catheter includes a catheter shaft having a cut that extends longitudinally between the proximal end and the distal end of the catheter and that extends radially from the catheter shaft outer surface to the guidewire lumen. A guide member slidably coupled to the catheter shaft functions to open the cut such that the guidewire may extend transversely into or out of the cut at any location along its length. By moving the guide member, the effective over-the-wire length of the otw/sw catheter is adjustable.

When using the otw/sw catheter, the guidewire is maneuvered through the patient's vascular system such that the distal end of the guidewire is positioned across the treatment site. With the guide member positioned near the distal end of the catheter, the proximal end of the guidewire is threaded into the guidewire lumen opening at the distal end of the catheter and through the guide member such that the proximal end of the guidewire protrudes out of the proximal end of the guide member. By securing the guide member and the proximal end of the guidewire in a fixed position, the catheter may then be transported over the guidewire by advancing the catheter toward the guide member. In doing so, the catheter advances through the guide member such that the guidewire lumen envelops the guidewire as the catheter is advanced into the patient's vasculature. In a PTCA embodiment, the otw/sw catheter may be advanced over the guidewire in this manner until the distal end of the catheter having the dilatation balloon is positioned within the stenosis and essentially the entire length of the guidewire is encompassed within the guidewire lumen.

Furthermore, the indwelling otw/sw catheter may be exchanged with another catheter by reversing the operation described above. To this end, the indwelling catheter may be removed by withdrawing the proximal end of the catheter from the patient while holding the proximal end of the guidewire and the guide member in a fixed position. When the catheter has been withdrawn to the point where the distal end of the cut has reached the guide member, the distal portion of the catheter over the guidewire is of a sufficiently short length that the catheter may be drawn over the proximal end of the guidewire without releasing control of the guidewire or disturbing its position within the patient. After the catheter has been removed, another otw/sw catheter may be threaded onto the guidewire and advanced over the guidewire in the same manner described above with regard to the otw/sw catheter. The otw/sw catheter also allows a guidewire to be removed from an indwelling catheter and re-inserted or exchanged without having to withdraw the catheter from the patient. Thus, the otw/sw catheter overcomes these and many of the other difficulties discussed in association with RX catheters.

Despite these advantages, original otw/sw catheters in accordance with the '356 patent had difficulties related to movement of the guidewire through the guide member. As disclosed in the '356 patent, the use of a hypodermic tubing member to direct a guidewire into and out of the guidewire lumen was found to be effective while the guidewire was stationary within the guide member, and while the catheter was translocated there through. FIG. 8 illustrates the problem encountered when the guidewire was withdrawn through the guide member. The hypodermic tubing member, designated 26, would often scrape pieces of a lubricious coating from the guidewire, and the resulting shavings, designated generally as 16, would become jammed in the annular space between guidewire 15 and hypodermic tubing member 26, preventing further movement of the guidewire.

In a more significant problem with the original otw/sw catheter, it could fail to adequately contain the guidewire within the guidewire lumen during normal operation. In particular, as the catheter was advanced over the guidewire, the catheter could bend or buckle such that the guidewire could protrude from the catheter shaft. If the guidewire protruded from the catheter shaft, it could subsequently become pinched, and the distal end of the guidewire could be pulled out of or pushed beyond the treatment site, thus complicating the procedure and requiring repositioning within the patient's vasculature. Bending or buckling of a otw/sw catheter could also occur proximal to the guide member, where the guidewire is absent from the guidewire lumen. It is among the general objects of the invention to provide an improved device that overcomes the foregoing difficulties.

### SUMMARY OF THE INVENTION

The present invention is an over-the-wire catheter having a guidewire lumen with a guide way extending along a shaft portion, and a slidable instrument disposed about the shaft portion and extending transversely through the guide way for surrounding and selectively gripping a guidewire disposed in the guidewire lumen. The slidable instrument may be operated to control the location of the guidewire within a patient.

### BRIEF DESCRIPTION OF THE DRAWINGS

These and other features, aspects and advantages of the present invention will become better understood with reference to the following description, appended claims, and accompanying drawings where:

FIG. 1 is a longitudinal sectional illustration of a portion of a prior art coaxial over-the-wire catheter and guidewire system;

FIG. 2 is a transverse sectional illustration of a coaxial prior art over-the-wire catheter and guidewire system, taken along the line 2--2 of FIG. 1;

FIG. 3 is a longitudinal sectional illustration of a portion of a prior art multi lumen over-the-wire catheter and guidewire system;

FIG. 4 is a transverse sectional illustration of a multi lumen prior art over-the-wire catheter and guidewire system, taken along the line 4--4 of FIG. 3;

FIG. 5 is a longitudinal sectional illustration of a portion of a prior art rapid exchange catheter and guidewire system;

FIG. 6 is a transverse sectional illustration of a prior art rapid exchange catheter and guidewire system, taken along the line 6--6 of FIG. 5;

FIG. 7 is partial longitudinal sectional illustration of a portion of a prior art rapid exchange catheter and guidewire system, shown within a guiding catheter;

FIG. 8 is a partial longitudinal sectional illustration of a portion of a prior art otw/sw catheter and guidewire system;

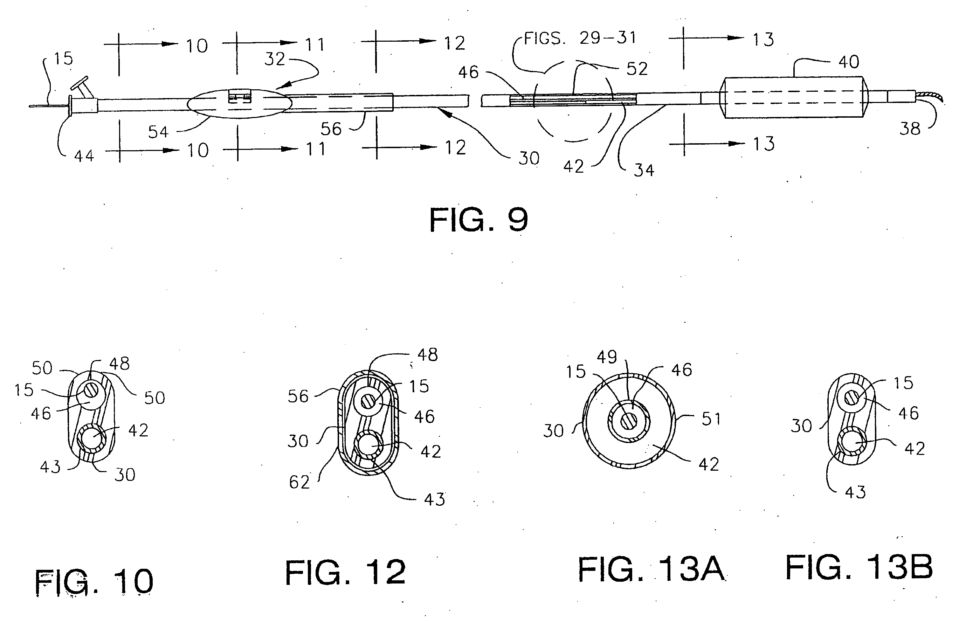

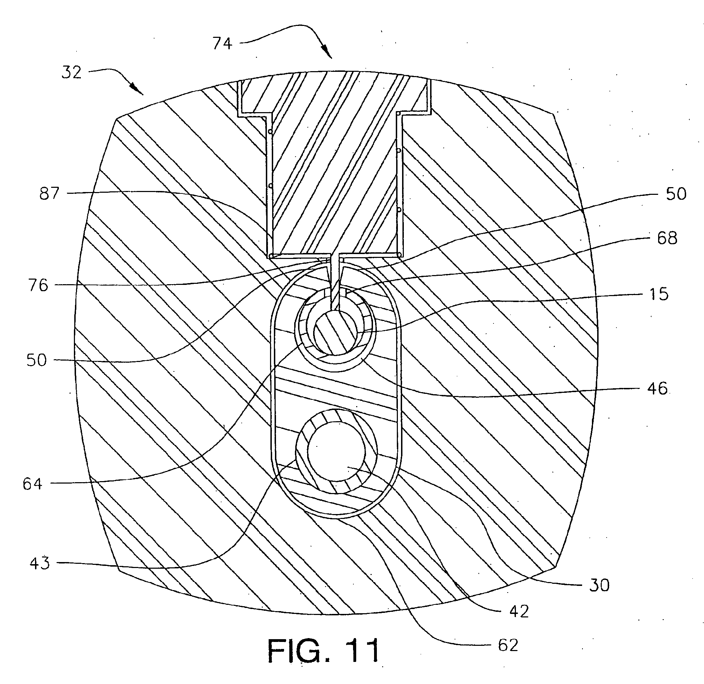

FIG. 9 is an elevation view of a catheter, a guidewire and a sliding instrument in accordance with the present invention;

FIG. 10 is a transverse sectional illustration of the catheter and guidewire as seen along the line 10--10 of FIG. 9;

FIG. 11 is a transverse sectional illustration of the catheter, guidewire and sliding instrument as seen along the line 11--11 of FIG. 9;

FIG. 12 is a transverse sectional illustration of the catheter, sliding instrument and guidewire as seen along the line 12--12 of FIG. 9;

FIG. 13A is a transverse sectional illustration of the catheter and guidewire as seen along the line 13--13 of FIG. 9;

FIG. 13B is a multi lumen alternative embodiment of the catheter and guidewire depicted in FIG. 13;

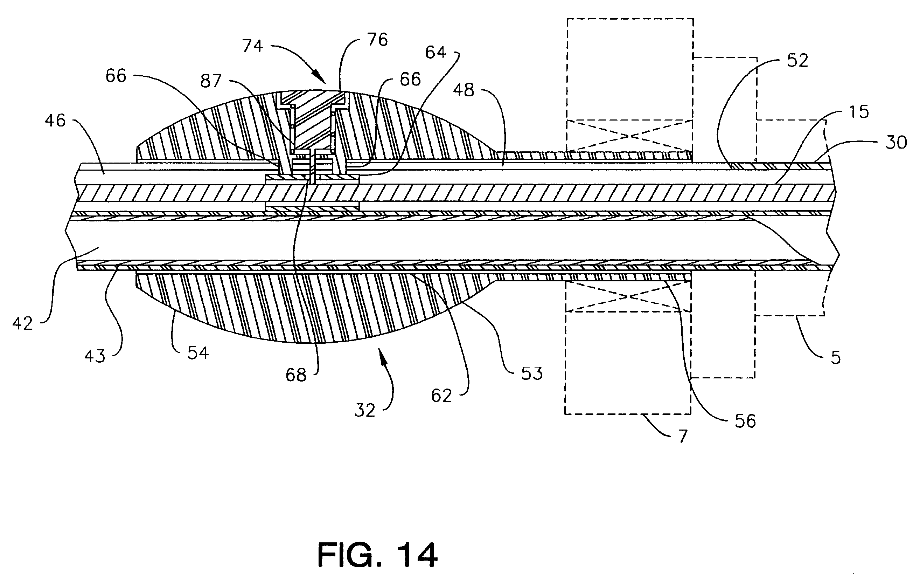

FIG. 14 is a longitudinal sectional illustration of a portion of a catheter, a guidewire and a sliding instrument in accordance with the present invention, with a guiding catheter and a Tuohy-Borst fitting shown schematically,;

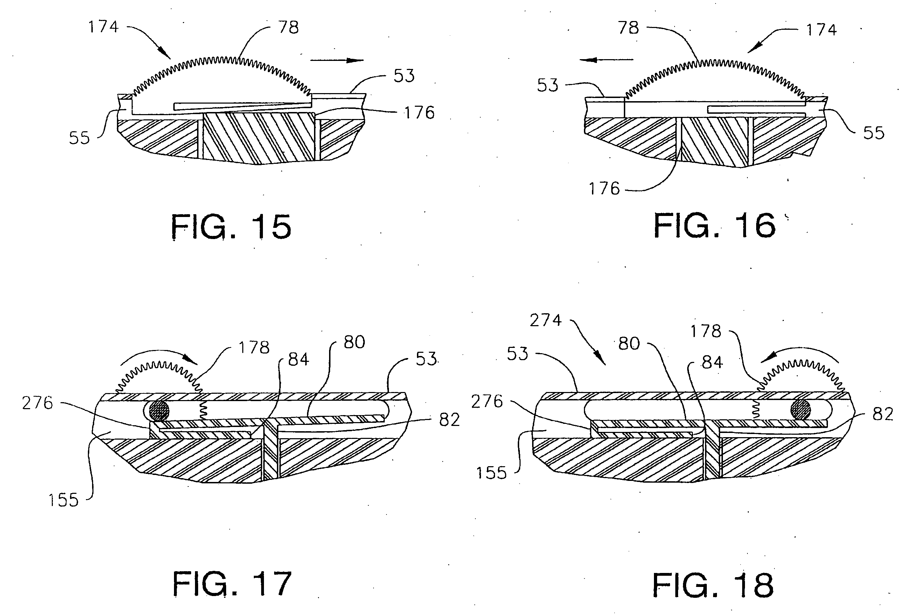

FIG. 15 is a sectional illustration of a portion of a first alternative clamp mechanism in accordance with the present invention;

FIG. 16 is a sectional illustration of the portion of the first alternative clamp mechanism of FIG. 15, shown in an alternate position;

FIG. 17 is a sectional illustration of a portion of a second alternative clamp mechanism in accordance with the present invention;

FIG. 18 is a sectional illustration of the portion of the second alternative clamp mechanism of FIG. 17, shown in an alternate position;

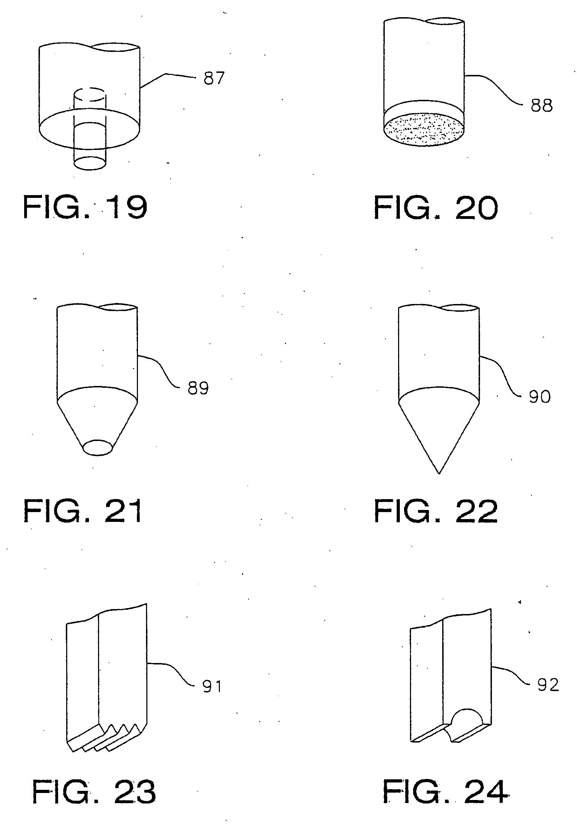

FIGS. 19-24 illustrate modified forms of the internal, gripping end of a clamp member in accordance with the present invention;

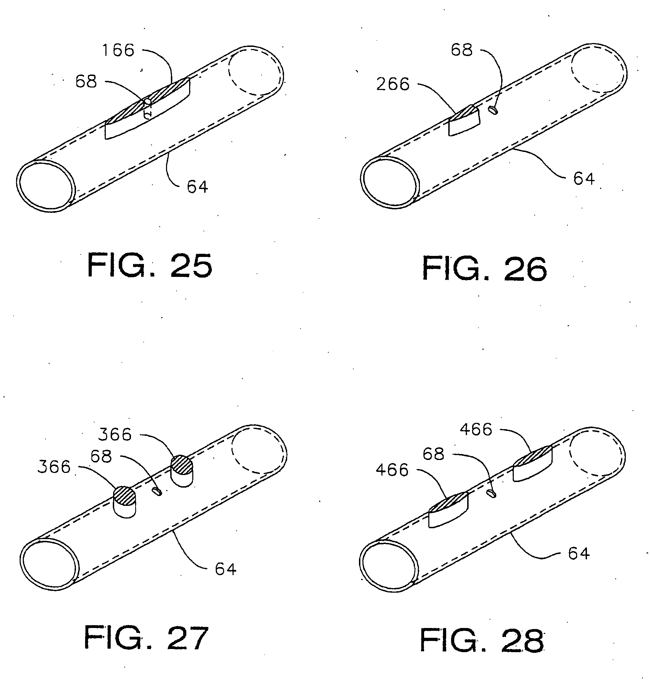

FIGS. 25-28 illustrate modified forms of the tubular receiver and stanchions in accordance with the present invention;

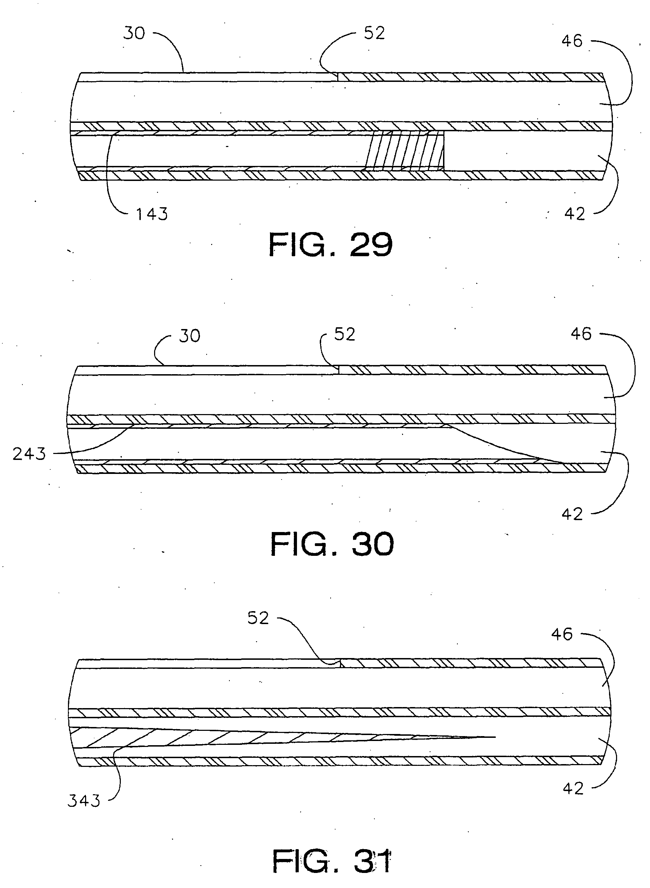

FIG. 29 is an enlarged view of a partially sectioned portion of the catheter in FIG. 9, showing the distal end of the stiffening member;

FIG. 30 illustrates a modified form of the distal end of the stiffening member depicted in FIG. 29; and

FIG. 31 is partial longitudinal sectional illustration of a portion of a prior art rapid exchange catheter and guidewire system, shown within a guiding catheter and illustrating a modified form of the stiffening member.

The drawings are not to scale.

### DETAILED DESCRIPTION OF THE INVENTION

As shown in FIG. 9, the invention includes a catheter, indicated generally by the reference character 30 on which sliding instrument 32 is slidably mounted. Guidewire 15 is illustrated as extending through catheter 30 and as being contained and housed within catheter 30 except for the distal end of guidewire 15, which may protrude distally out of distal opening 38 of catheter 30, and the proximal end of guidewire 15, which may protrude proximally out of fitting 44.

Catheter 30 includes an elongate, flexible, cylindrical shaft, which may be formed from an extruded plastic material such as, for example, polyethylene or polyethylene block amide (PEBA) copolymer. In the embodiment shown in FIG. 9, catheter 30 is a PTCA catheter having balloon 40 mounted around the catheter shaft near the distal end of catheter 30. Balloon 40 may be inflated and deflated through inflation lumen 42 formed through the shaft of the catheter 30, as shown in FIGS. 10-13A and 13B. Inflation lumen 42 communicates with fitting 44 and extends the length of catheter 30 to terminate in communication with the interior of balloon 40. Fitting 44 is intended to be connected to a suitable source of pressurized fluid or a partial vacuum (not shown) to inflate or deflate balloon 40. Catheter 30 also includes guidewire lumen 46, which communicates with fitting 44 and extends the length of catheter 30 to terminate at distal opening 38.

The shaft of catheter 30 is formed with longitudinal guide way 48, which, when catheter 30 is viewed in cross-section, as in FIG. 10, may be considered as defining a pair of flaps 50 which normally close together at guide way 48 to define enclosed guidewire lumen 46. The cross-section of guidewire lumen 46 may be circular or non-circular; in either case, the cross-sectional dimensions of guidewire lumen 46 are greater than the cross-sectional dimension of guidewire 15 to permit relative longitudinal movement between guidewire 15 and catheter 30. Optionally, inflation lumen 42 encompasses elongate stiffening member 43, which can cause the shaft of catheter 30 to have greater bending stiffness than guidewire 15. Stiffening member 43 may extend at least through the proximal segment of catheter 30 that includes guide way 48, thus preventing the shaft from bending such that guide way 48 could buckle, allowing guidewire 15 to protrude from the catheter shaft, as discussed earlier with respect to the original otw/sw catheter.

The proximal end of guide way 48 may terminate at or near fitting 44. In the embodiment shown in FIGS. 9 and 14, distal end 52 of guide way 48 terminates short of distal end 38 of catheter 30, thereby leaving uncut distal segment 34 of catheter 30. Adjacent guide way distal end 52, the shaft of catheter 30 may transform from the more proximal side-by-side arrangement of lumens to a more distal coaxial arrangement, as will be understood by those of skill in the art. Distal segment 34 may comprise a coaxial arrangement of two tubes, as shown in FIG. 13A, with inner tube 49 communicating with and surrounding an extension of guidewire lumen 46. Outer tube 51 encompasses inner tube 49, forming an annular lumen that extends inflation lumen 42 from the region of guide way distal end 52 to balloon 40. Alternatively, distal segment 34 may comprise a side-by-side arrangement of the inflation lumen 42 and guidewire lumen 46 as shown in FIG. 13B.

Sliding instrument 32 comprises body 53 having proximal and distal ends, 54, 56, respectively, as shown in FIGS. 9 and 14. Passageway 62 extends longitudinally in a generally straight line from body proximal end 54 to body distal end 56. Tubular receiver 64 is mounted within passageway 62 on one or more stanchions 66 and tubular receiver 64 is sized to slidably receive guidewire 15. Passageway 62 slidably receives the catheter shaft portion that includes guide way 48 such that guidewire lumen 46 slides over tubular receiver 64, as illustrated in FIG. 11. Tubular receiver 64 has side opening 68 radially aligned within passageway 62 to receive clamp member 76 of clamp mechanism 74. Stanchion 66 may serve to align catheter 30 within catheter passageway 62, and especially to line up guide way 48 with receiver side opening 68 and clamp member 76. Stanchion 66 may be fin-shaped and may surround receiver side opening 68 or be disposed adjacent thereto, as will be described below.

Manually operable clamp mechanism 74 is disposed within body 53, and includes clamp member 76, which moveably extends transversely into passageway 62, through guide way 48 and receiver side opening 68 to impinge against guidewire 15. Force applied by clamp mechanism 74 causes guidewire 15 to be gripped between clamp member 76 and an opposing interior wall of tubular receiver 64. When gripping force is released, clamp member 76 retracts from impingement against guidewire 15 to permit free-sliding movement of guidewire 15 through tubular receiver 64. In the example depicted in FIGS. 11 and 14, clamp member 76 comprises a sliding pin having an exposed external end that may be manually depressed against a coil spring to cause the internal end of the pin to impinge against guidewire 15. Upon release of the manual force, the coil spring will retract clamp member 76 from impingement against guidewire 15. The following embodiments are two examples among numerous possible alternative configurations of clamp mechanism 74.

FIGS. 15 and 16 illustrate a first alternative clamp mechanism 174, wherein cam or sliding wedge 78 can be manually operated to traverse channel 55 within body 53, such that sliding wedge 78 will depress or release the external end of clamp member 176. Upon release of clamp member 176 by sliding wedge 78, a spring-like element (not shown) can retract clamp member 176 from impingement against guidewire 15. FIGS. 17 and 18 illustrate a second alternative clamp mechanism 274, wherein clamp member 276 comprises platen 80, which is integrally molded across the external end of sliding pin 82. Leaf spring 84 is also integrally molded in clamp member 276 at an angle to platen 80, and leaf spring 84 can be affixed to or disposed against body 53 to effect retraction of clamp member 276 from its impingement against guidewire 15. Roller 178 can be manually rolled along platen 80, which is disposed in slotted channel 155 of body 53, thereby depressing or releasing the external end of sliding pin 82. Platen 80 can be shaped to use the force applied by roller 178 like a lever acting on sliding pin 82. Unlike self-releasing clamp mechanism 74 described above, both clamp mechanisms 174 and 274 are capable of remaining in any selected position to lock or unlock their grip on guidewire 15. Other alternative clamp mechanisms may incorporate screw-threaded knobs or wheels, or various combinations of the mechanisms described herein.

Components making up sliding instrument 32, including body 53, clamp mechanism 74, tubular receiver 64 and stanchion 66, may be molded from a suitable rigid plastic material, such as nylon or nylon-based co-polymers that are preferably lubricious. Alternatively, sliding instrument 32 may be made of a suitable metal such as stainless steel, or sliding instrument 32 may have both metal components and plastic components. For ease in manufacturing, sliding instrument 32 may be comprised of molded parts that snap-fit together to form the final configuration. Furthermore, tubular receiver 64 may be fabricated from metal hypotubing, which can be affixed to the inner ends of one or more metal stanchions 66 by suitable techniques such as soldering, welding or brazing. In such an example, the outer ends of stanchions 66 can be insert molded into body 53.

Clamp members 76, 176 and sliding pin 82 may have impingement element 86 disposed at an internal end and being adapted for gripping engagement with guidewire 15. One form of impingement element 86 is a square-cut end. FIGS. 11, 14 and 19 show first alternate impingement element 87 having a tip that is stepped-down in diameter. The form of impingement element 87 provides a sturdy shaft for clamp members 76, 176 and a reduced diameter tip portion sized and shaped to extend through guide way 48 and receiver side opening 68. Impingement element 87 may comprise a pin of a hard material such as a metal or a ceramic that is insert molded into the end of a plastic shaft. FIG. 20 shows second alternate impingement element 88 having a tip with an enhanced gripping surface, such as a rough-textured surface, an abrasive surface, or a tacky adhesive surface. FIG. 21 shows third alternate impingement element 89 comprising a tapered pin, which reduces the contact area between clamp members 76, 176 and guidewire 15. FIG. 22 shows fourth alternate impingement element 90 having a tapered point, which further focuses the contact force between clamp members 76, 176 and guidewire 15. FIG. 23 shows fifth alternate impingement element 91 comprising a serrated tip. Impingement element 91 has a shaft cross-section that is square or another non-circular shape such that a correspondingly shaped bore in body 53 can maintain alignment of the tip serrations transverse to the axis of guidewire 15. A single chisel point (not shown) may also be used instead of the serrations of impingement element 91. FIG. 24 shows sixth alternate impingement element 92 comprising a notched end. Impingement element 92 also has a non-circular shaft cross-section to maintain alignment of the notched end with the axis of guidewire 15.

Catheter 30 extends through passageway 62 in body 53, engaging stanchion 66 and clamp member 76, which extend transversely through guide way 48 in catheter 30 to spread flaps 50 apart. Except for the portion of catheter 30 that engages stanchion 66 and clamp member 76, flaps 50 remain drawn together under the influence of the inherent resiliency of the catheter shaft to close guide way 48, thus enclosing guidewire 15 within guidewire lumen 46. Catheter 30 slides over tubular receiver 64, and guidewire 15 slides through tubular receiver 64 unless guidewire 15 is being selectively gripped by clamp mechanism 74. Optional stiffening member 43 can avert buckling of catheter 30 by providing shaft rigidity that is especially advantageous when the catheter is being pushed into sliding instrument proximal end 54. Guidewire 15 may be inserted into guidewire lumen 46 and tubular receiver 64, either by back-loading guidewire 15 through distal opening 38 or by front-loading guidewire 15 through fitting 44. Guidewire 15 can be removed through fitting 44 and can be replaced or exchanged for another guidewire while catheter 30 is indwelling in a patient.

While guidewire 15 is being selectively gripped by clamp mechanism 74, guidewire 15 is held in a fixed position relative to sliding instrument 32, and catheter 30 can be translocated over guidewire 15 and through sliding instrument 32. Alternatively, longitudinal movement of sliding instrument 32 can slide guidewire 15 through guidewire lumen 46 while catheter 30 is held in a fixed position. To insert catheter 30 into a patient, guidewire 15 is maneuvered through the patient's vascular system such that the distal end of guidewire 15 is positioned across the treatment site. With sliding instrument 32 positioned near the distal end of catheter 30, the proximal end of guidewire 15 is back-loaded into distal opening 38 and through sliding instrument 32. By gripping sliding instrument 32 and guidewire 15 in a fixed position, catheter 30 may then be transported over guidewire 15 by advancing the proximal segment of catheter 30 distally toward sliding instrument 32. In doing so, catheter 30 advances through sliding instrument 32 such that guidewire lumen 46 envelops guidewire 15 as catheter 30 is advanced into the patient's vasculature. Catheter 30 may be advanced over guidewire 15 in this manner until the distal end of catheter 30 having balloon 40 is positioned within the stenosis and substantially the entire length of guidewire 15 is encompassed within guidewire lumen 46.

Any portion of guidewire 15 may be selectively gripped by clamp mechanism 74. To remove catheter 30 over guidewire 15 that is indwelling in a patient, clamp mechanism 74 is engaged to grip a portion of guidewire 15 that is extending proximally from guiding catheter 5. Thus, guidewire 15 can be manually restrained within guide catheter 5 by sliding instrument 32. Optionally, sliding instrument 32 may also be secured within Touhy-Borst fitting 7, which is attached to guiding catheter 5 such that guidewire 15 can be mechanically restrained within guide catheter 5, as shown schematically in FIG. 14. Sliding instrument distal end 56 may be configured to surround passageway 62 and to be received in Touhy-Borst fitting 7. While guidewire 15 is restrained within guide catheter 5, the shaft of catheter 30 can be withdrawn over guidewire 15 until guide way distal end 52 abuts stanchion 66 or clamp member 76. Then, clamp mechanism 74 can be operated to release its grip on guidewire 15 such that catheter 30 and sliding instrument 32 can be slid off of guidewire 15, which remains in position within the patient. Another catheter, such as catheter 30, can be inserted into the patient by reversing the above steps.

As illustrated in FIGS. 10, 11 and 12, at least the shaft portion of catheter 30 comprising guide way 48 is generally oval in cross-sectional shape, which minimizes the amount of material surrounding guidewire lumen 46 and inflation lumen 42. One advantage of such a catheter shape is that the small perimeter, and the correspondingly small area of the cross-section will maximize the surrounding annular space when catheter 30 lies within guiding catheter 5. An additional advantage of the oval cross-sectional shape is that catheter 30 will tend to align itself with catheter passageway 62, which has a matching oval cross-section, as shown in FIGS. 11 and 12. However, proximal shaft section 35 and catheter passageway 62 may also be generally circular. Figure 13A depicts distal section 34 of catheter 30 having a round cross-sectional shape and a coaxial arrangement of inner tube 49 and outer tube 51. Optionally, catheter distal section 34 can have an oval cross section regardless of whether there is a multi lumen arrangement, as shown in FIG. 13B, or a coaxial layout of the guidewire and inflation lumens. Because tubular receiver 64 is generally constrained within guidewire lumen 46 by flaps 50, body 53 does not need to completely surround catheter 30. Instead, a modified form (not shown) of body 53 can partially surround catheter 30 and the inherent resilience of flaps 50 over tubular receiver 64 may be relied upon to hold body 53 and catheter 30 together.

FIGS. 25-28 illustrate modified forms of tubular receiver 64 and stanchions in accordance with the present invention. All stanchions are illustrated as having been sectioned away from body 53. FIG. 25 shows tubular receiver 64 having fin-shaped stanchion 166 with side opening 68 extending transversely there through. FIG. 26 shows tubular receiver 64 having stanchion 266 located adjacent to side opening 68. The edge of stanchion 266 adjacent to side opening 68 is squared off, and the other edge is fin shaped. FIG. 27 shows tubular receiver 64 having two stanchions 366 located adjacent to side opening 68. Both stanchions 366 are cylindrical in shape. FIG. 28 shows tubular receiver 64 having two stanchions 466 located adjacent to side opening 68. Both stanchions 466 are fin shaped. Other modified forms are also possible.

FIGS. 29-31 show modified forms of the stiffening member in accordance with the invention. FIG. 29 illustrates tubular stiffening member 143, a modified form wherein the distal end is spirally cut to provide more gradual transition in flexibility from the stiffened portion to the unstiffened portion of catheter 30. FIG. 30 illustrates tubular stiffening member 243, another modified form wherein the distal end is skived, or cut at an angle to also accomplish a gradual transition in flexibility. Spirally cut stiffening member 143 may also have a skived distal end. FIG. 31 illustrates mandrel-type stiffening member 343, another modified form having a tapered distal end and being disposed within inflation lumen 42, leaving sufficient annular space for fluid flow. While the stiffening member is shown as a component within the catheter shaft, a reinforced catheter wall is also contemplated if it provides sufficient support.

In examples where the catheter shaft of the invention incorporates tubular stiffening members 43, 143 or 243, it is advantageous to fit the tubular member tightly within inflation lumen 42, such that all of the inflation/deflation fluid will flow through the lumen of the tubular stiffening member. The desired tight fit can be achieved by over-extruding the polymer shaft of catheter 30 onto the tubular member. Having the stiffening member tightly fitted within the catheter shaft also helps it to resist twisting and kinking. In a first method of manufacturing such a catheter shaft, a substantial length of metal tubing can be fed through a wire-coating type of polymer extrusion head. Next, the substantial length of plastic-jacketed tubing thus formed can be cut into approximately catheter-length pieces. In order to modify the distal ends of tubular members 143 or 243, a distal section of the over-extruded plastic jacket is cut away, exposing the metal tubing for alteration, such as spiral cutting or skiving. The last step in forming the shaft of catheter 30 is to add an uncut distal portion 34, as by adhesive or by thermoplastic welding, using heat-shrink tubing and temporary support mandrels in guidewire lumen 46 and inflation lumen 42.

Alternatively, tubular members 43, 143 or 243 having a finished length and the desired tip configuration can be fed, one-at-a-time, through a wire-coating type of polymer extrusion head. A distal section of the over-extruded plastic jacket is cut away and an uncut distal portion 34 can be added.

While the invention has been particularly shown and described with reference to the preferred embodiments thereof, it will be understood by those skilled in the art that various changes in form and detail may be made there in without departing from the spirit and scope of the invention.

## Claims

1. An instrument capable of selectively gripping an elongate guidewire disposed in a lumen of a catheter, wherein the catheter has a guide way extending longitudinally between catheter proximal and distal ends and extending radially from a catheter outer surface to the lumen, the instrument comprising:
a tubular receiver capable of being disposed within the lumen and surrounding the guidewire therein, the receiver being axially slidable with respect to both the lumen and the guidewire and having a side opening that is radially alignable with the guide way;
a body capable of sliding along the catheter outer surface, the body being connected to the tubular receiver by at least one stanchion capable of spreading the guide way and extending transversely there through; and
a clamp mechanism associated with the body and having a clamp member capable of moveably extending transversely through the guide way and the receiver side opening to impinge against the guidewire, thereby selectively gripping the guidewire between the clamp member and an opposing interior wall of the tubular receiver.

2. The instrument of claim 1, wherein the at least one stanchion is located adjacent to and aligned longitudinally with the receiver side opening.

3. The instrument of claim 1, wherein the at least one stanchion comprises two stanchions located on opposite sides of the receiver side opening.

4. The instrument of claim 3, wherein the two stanchions are fin-shaped such that a long transverse axis of each stanchion is aligned with the side opening.

5. The instrument of claim 1, wherein the receiver side opening extends transversely through the at least one stanchion.

6. The instrument of claim 5, wherein the at least one stanchion is fin-shaped such that a long transverse axis of the at least one stanchion is aligned with the side opening.

7. The instrument of claim 1, wherein the body is adapted to surround, circumferentially, at least a portion of the catheter.

8. The instrument of claim 7, wherein at least a section of the body is adapted to completely surround the catheter circumferentially.

9. The instrument of claim 7, wherein a section of the body is adapted to completely surround the catheter circumferentially and to be inserted into a Tuohy-Borst fitting.

10. The instrument of claim 1, wherein the clamp member is retracted from impingement against the guidewire when the clamp mechanism is not selectively gripping the guidewire.

11. The instrument of claim 10, wherein the clamp member is retracted from impingement against the guidewire by a spring or by shape memory of the clamp member itself.

12. The instrument of claim 10, wherein the clamp member is a sliding pin arranged perpendicular to the tubular receiver and having an impingement element disposed at an internal end, the impingement element being adapted for gripping engagement with the guidewire and being selected from a group consisting of cylindrical pins, tapered pins, metal pins, filled polymer pins, ceramic pins, tapered points, chisel points, notched ends, rough-textured surfaces, serrated surfaces, abrasive surfaces, tacky adhesive surfaces, and combinations of the above.

13. The instrument of claim 12, wherein the sliding pin has an external end exposed on the sliding body for manual actuation.

14. The instrument of claim 12, wherein the sliding pin has an external end arranged for actuation by a manual operator selected from a group consisting of sliding cams, sliding wedges, levers, roller clamps, screw-threaded wheels, screw-threaded knobs and combinations of the above.

15. The instrument of claim 1, wherein the clamp mechanism may be manually locked and unlocked to selectively grip the guidewire.
